# EUROPEAN PATENT APPLICATION

(11) **EP 2 474 614 A2**
(43) Date of publication of application: **11.07.2012**
(21) Application number: 10812255.7
(22) Date of filing: 25.08.2010
(51) Int. Cl.: C12N 15/11, C12N 15/63, C12N 15/66

(54) **DNA FRAGMENT FOR IMPROVING TRANSLATION EFFICIENCY, AND RECOMBINANT VECTOR CONTAINING SAME**

(30) Priority: 31.08.2009 KR 20090081403
(71) Applicant: Helix, Co., Limited, Kyeongsangbuk-do 790-784 (KR)
(72) Inventor: NA, Yun Jeong, Pohang Kyeongsangbuk-do 790-751 (KR); JEON, Eun Hyun, Pohang Kyeongsangbuk-do 790-390 (KR); KWON, Eun Hye, Pohang Kyeongsangbuk-do 790-751 (KR); KIM, Yong Woo, Jeju-do 690-802 (KR); SOHN, Eun Ju, Pohang Kyeongsangbuk-do 790-751 (KR); HWANG, In Hwan, Pohang Kyeongsangbuk-do 790-751 (KR)
(74) Representative: Leathley, Anna Elisabeth
(86) International application number: PCT/KR2010/005669
(87) International publication number: WO 2011/025242

(57) **Abstract**

The present invention relates to a DNA fragment for improving translation efficiency, and a recombinant vector containing the same, and more specifically, to a DNA fragment which comprises any one nucleotide sequence selected from the group consisting of SEQ ID NOs: 1-6, SEQ ID NOs: 8-10, SEQ ID NOs: 13 and 14 and SEQ ID NO: 16, and improves the translation efficiency of a heterologous protein placed in the downstream, and a recombinant vector containing the DNA fragment. The DNA fragment for improving translation efficiency according to the present invention and a recombinant vector containing the same can improve the translation of a heterologous protein in a transgenic plant. In addition, if a leader polynucleotide inducing the targeting to a particular cellular organelle of a plant is further linked to the recombinant vector in an operable manner, the heterologous protein is targeted to the specific cellular organelle and can be stably accumulated, thereby enabling the mass production of the heterologous protein from the plant.

## Description

This application claims priority to Korean Patent Application No. 10-2009-0081403 filed on August 31, 2009 and all the benefits accruing therefrom under 35 U.S.C. §119, the contents of which are incorporated by reference in their entirety.

### TECHNICAL FIELD

The present invention relates to a DNA fragment for improving translation efficiency, and a recombinant vector containing the same, thereby enabling the mass production of a heterologous protein from a plant.

### BACKGROUND ART

In general, plants have high potential for producing biopharmaceutical protein and peptides, since they are easy to transform and economical to be used as protein material. To date, most of biopharmaceuticals are produced by transforming mammalian cells, bacteria and fungus (Ganz PR *et al*., 1996; Ma JK *et al.,* 1999; Pen J, 1996). Producing therapeutic proteins using plants instead of mammalian cells, bacteria or fungus have several advantages in economical and in quality aspects: it reduces the risk of contamination by pathogenic bacteria, increases the production yield, can be produced in seed or other storage organs. In addition, plants have a great potential for commercial biopharmaceutical production since they are economical sources for producing recombinant products, cultivation, harvest, storage and treatment of the transgenic crops can utilize the current infrastructure, and requires relatively low cost in investment.

Therefore, the development of a plant expression system by transforming a plant cell to produce a useful recombinant heterologous protein with high efficiency is greatly desired. The plant expression system is appealing, since the expression level of the recombinant heterologous protein can be increased by using the innate sorting and targeting mechanism used for targeting host proteins into cellular organelles, and it also has an advantage for the large-scale production of plant-derived biopharmaceuticals.

When using plant systems for producing useful recombinant heterologous protein, selection of species, tissue, expression and recovery strategy, and post-translational processing are important factors in determining the possibility of plant-based production. Recently, there have been many studies on producing useful proteins from plants by transformation of the nucleus.

However, there are limitations when producing useful proteins on a large-scale because only one or a small number of genes are transferred into the nucleus. In addition, there are some problems that the transgene is not expressed when it is inserted into a heterochromatin region even though it is transferred or the level of transduction may vary depending on the inserted site. Various approaches have been made to transfer genes into mitochondria, chloroplast, or chromatophore to induce large-scale expression.

In eukaryotes, the protein expression is primarily regulated by transcriptional initiation at the messenger RNA level, by the stability of the genetic information transcribed from DNA to messenger RNA, by the transcript processing and modification, and secondarily, by translational initiation at the protein level and by regulation of the protein stability.

Researchers have been focusing on increasing the production of useful heterologous protein by regulating the expression of the heterologous protein after transforming a gene encoding the heterologous protein into a cell. Strong promoters were developed to increase the protein synthesis in early processing, in particular, during the transcription step. In order to express the target gene efficiently in the transgenic plant, several factors such as the choice of promoters, and introns, and codon usage must be considered. It can be harmful to the transgenic plant system when the amount of gene transcript is increased too much. There is a problem of limitation in the amount of transcription can be increased. Moreover, there are reports indicating that most of the mRNA produced by transcription is not translated into proteins.

Regulation at the translation step is known to have more effect on the final amount of protein synthesized from a gene, as compared to regulation at the transcription step. In order to produce the useful heterologous proteins in large-scale, regulation of protein expression at the translation step rather than at the transcription step, and new protein expression regulating techniques that targets the heterologous protein to specific cellular organelles; are required.

The present inventors carried out extensive research to develop a method for producing a useful heterologous protein from plants on a large-scale by increasing the protein synthesis at the translation level. As a result, a DNA fragment with high translation efficiency was discovered from *A. thaliana* genomic DNA. When a plant was transformed with a recombinant vector containing the DNA fragment or with a recombinant vector containing a leader polynucleotide for targeting a polypeptide to ER or chloroplast operably linked to the DNA fragment, the translation efficiency of the heterologous protein was increased. Also, it is confirmed that it is possible to mass produce the heterologous protein by sorting and stable accumulation of the heterologous protein to ER or chloroplast using the recombinant vector, thereby the present inventors completed the present invention.

### DISCLOSURE OF THE INVENTION

### TECHNICAL PROBLEM

Accordingly, it is an object of the present invention to provide a DNA fragment for improving translation efficiency of a protein.

It is another object of the present invention to provide a recombinant vector containing the DNA fragment.

It is still another object of the present invention to provide a cell or a plant transformed with the recombinant vector.

It is another object of the present invention to provide a method for mass producing a heterologous protein from a plant comprising a step of introducing the recombinant vector into the plant.

### TECHNICAL SOLUTION

To achieve the above objectives, the present invention provides a DNA fragment for improving translation efficiency of a heterologous protein placed in the downstream, which comprises a polynucleotide having any one nucleotide sequence selected from the group consisting of SEQ ID NOs: 1∼6, SEQ ID NOs: 8∼10, SEQ ID NOs: 13∼14 and SEQ ID NO: 16.

Also, the present invention provides the recombinant vector containing the DNA, which comprises a polynucleotide having any one nucleotide sequence selected from the group consisting of SEQ ID NOs: 1∼6, SEQ ID NOs: 8∼10, SEQ ID NO: 13 and SEQ ID NO: 14.

In an aspect of the recombinant vector of the present invention, the DNA fragment may operably linked to a promoter, and a polynucleotide encoding the heterologous protein.

In another aspect of the recombinant vector of the present invention, the DNA fragment may be operably linked to an additional polynucleotide sequence which targets and retains the heterologous protein to ER of a plant cell.

In still another aspect of the recombinant vector of the present invention, the polynucleotide sequence for targeting the heterologous protein to ER may be a polynucleotide encoding BiP (chaperone binding protein). The polynucleotide encoding BiP may have nucleotide sequence represented by SEQ ID NO: 18, and the polynucleotide sequence for retaining the heterologous protein to ER may be a polynucleotide encoding nucleotide sequence peptide HDEL (His-Asp-Glu-Leu) or KDEL (Lys-Asp-Glu-Leu).

In still another aspect of the present invention, the recombinant vector may additionally comprise a polynucleotide encoding cellulose-binding domain (CBD) in an operable manner. The polynucleotide encoding CBD may have nucleotide sequence represented by sequence SEQ ID NO: 21.

The present invention also provides a cell transformed with the recombinant vector.

The present invention also provides a transgenic plant transformed with the recombinant vector.

The present invention also provides a method for mass producing a heterologous protein, which comprises a step of introducing the recombinant vector into a plant.

In one aspect of the present invention, the introduction of the recombinant vector into a plant is performed using any one selected from the group consisting of *Agrobacterium sp*.-mediated transformation, particle gun bombardment, silicon carbide whiskers, sonication, electroporation and PEG (polyethylene glycol) precipitation.

In another aspect of the present invention, the plant is a dicotyledon or a monocotyledon.

In still another aspect of the present invention, the dicotyledon is selected from the group consisting of *A. thaliana,* soybean, tobacco plant, eggplant, red pepper, potato, tomato, Chinese cabbage, radish, cabbage, peach, pear, strawberry, watermelon, melon, cucumber; carrot and celery.

In another aspect of the invention, the monocotyledon is selected from the group consisting of rice, barley, wheat, rye, corn, sugar cane, oat and onion.

### ADVANTAGEOUS EFFECTS

As set forth above, the DNA fragment for improving translation efficiency according to the present invention and a recombinant vector containing the same can improve the translation efficiency of a heterologous protein in a transgenic plant. In addition when a leader polynucleotide targeting to a particular cellular organelles of a plant is further added to the recombinant vector in an operable manner, the heterologous protein is targeted to the organelles and can be stably accumulated therein, thereby enabling mass production of the heterologous protein from the plant.

### BRIEF DESCRIPTION OF THE DRAWINGS

FIG. 1a a diagram illustrating the recombinant vector 35Sp-UTR(screened SEQ ID NOs: 1∼50)::GFP for expressing the GFP fusion protein in the protoplast of A. *thaliana.*

FIG. 1b is a fluorescence microscope image showing the expression of GFP in the *A. thaliana* leaf after introducing plasmid 35Sp-UTR(screened UTR NOs: 1∼50)::GFP into A. *thaliana* protoplast by the PEG-mediated transformation method.

FIG. 1c is Western blot image showing the expression of GFP from the leaf of *A. thaliana* transformed with 35Sp-UTR(SEQ ID NOs: 1∼6)::GFP using GFP antibody. Lanes are as follows: lane 1, control RbcUTR; lane 2, 5'-UTR NO: 1 (SEQ ID NO: 1); lane 3, 5'-UTR NO: 2 (SEQ ID NO: 2); lane 4, 5'-UTR NO: 6 (SEQ ID NO: 3); lane 5, 5'-UTR NO: 7 (SEQ ID NO: 4); lane 6, 5'-UTR NO: 24 (SEQ ID: 5); and lane 7, 5'-UTR NO: 35 (SEQ ID NO: 6).

FIG. 2a is Western blot images showing the expression of GFP from the leaf of *A. thaliana* transformed with 35Sp-UTR(SEQ ID NOs: 7∼11)::GFP using GFP antibody. Lanes are as follows: lane 1, control RbcUTR; lane 2, SEQ ID NO: 1; lane 3, SEQ ID NO: 7; lane 4, SEQ ID NO: 8; lane 5, SEQ ID NO: 9; lane 6, SEQ ID: 10; and lane 7, SEQ ID NO: 11.

FIG. 2b is Western blot images showing the expression of GFP from the leaf of *A. thaliana* transformed with 35Sp-UTR(SEQ 1D NOs: 12∼15)::GFP using GFP antibody. Lanes are as follows: lane. 1, control RbcUTR; lane 2, SEQ ID NO: 1; lane 3, SEQ ID NO: 12; lane 4, SEQ ID NO: 13; lane 5, SEQ ID NO: 14; and lane 6, SEQ ID: 15.

FIG. 2c is Western blot images showing the expression of GFP from the leaf of *A. thaliana* transformed with 35Sp-UTR(SEQ ID NO: 16)::GFP using GFP antibody. Lane 1 represents control RbcUTR and lane 2 represents UTR SEQ ID NO: 16.

FIG. 3 is a series of immunoblot images for analyzing the expression of GFP using GFP antibody in a wheat germ extract system to investigate whether the DNA fragment for improving translation efficiency has an effect on increasing protein translation in monocotyledon.

FIG. 4 is a series of diagrams illustrating the recombinant vectors (35Sp-UTR35::BiP:GFP:HA and recombinant vector 35Sp-UTR35::BiP:GFP:HA:HDEL) containing the DNA fragment according to the present invention and signal sequence (Bip) for targeting to ER, or Bip sequence and HDEL sequence linked in an operable manner.

FIG. 5 is a series of fluorescence microscope images showing the expression of GFP in the ER after transforming *A. thaliana* with recombinant vector 35Sp-UTR35::BiP:GFP:HA (upper panel) wand recombinant vector 35Sp-UTR35::BiP:GFP:HA:HDEL (lower panel).

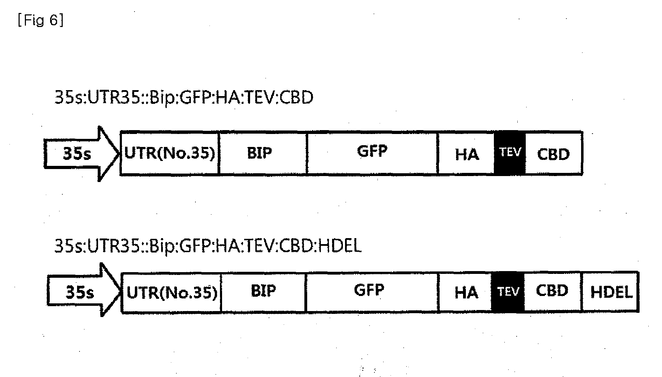

FIG. 6 is a series diagrams illustrating the recombinant vector 35Sp-UTR35::BiP:GFP:HA:TEV:CBD and recombinant vector 35Sp-UTR35::BiP:GFP:HA:TEV:CBD:HDEL containing CBD and TEV site linked in an operable manner to isolate the heterologous protein from the plant conveniently after targeting the protein to ER.

FIG. 7 is an immunoblot image showing the expression level of GFP at each time points using GPF antibody after transforming *A. thaliana* protoplast with recombinant vectors 35Sp-UTR35::BiP:GFP:HA:TEV:CBD and 35Sp-UTR35::BiP:GFP:HA:TEV:CBD:HDEL, respectively.

FIG. 8 is an immunoblot image of purified GFP from the *A, thaliana* protoplast transformed with 35Sp-UTR35::BiP:GFP:HA:TEV:CBD vector and isolated with CBD and TEV protease treatment.

FIG. 9 is a set of images of Coomassie-stained gel-after isolating and purifying the GFP protein with CBD, which was expressed in *A. thaliana* by transformed with 35Sp-UTR35::BiP:GFP:HA:TEV:CBD:HDEL vector.

FIG. 10a shows RT-PCR results using RNA isolated from the protoplast of the transgenic *A. thaliana* and GPF primers to investigate whether the DNA fragment for improving translation efficiency according to the invention can regulate the gene expression at the transcriptional level.

FIG. 10b is an immunoblot image showing the expression level of GFP from the protoplast of the transgenic *A. thaliana* to investigate whether the DNA fragment for improving translation efficiency according to the invention can regulate the gene expression at the translation level.

[45]

### BEST MODE FOR CARRYING OUT THE INVENTION

As a method for maximizing the production of the heterologous protein, the present inventors used the nucleotide sequence of 5'-untranslated region (5'-UTR) to control the protein expression at the translation level.

In general, the 5'-UTR of the mRNA is known to have important role in post-transcriptional regulation, regulation of the mRNA transport across the nucleus and regulation of protein translation efficiency and stability of the mRNA. The 5'-UTR, which is also known as the leader sequence, contains a ribosome binding site (RBS) called Shine-Dalgarno sequence (AGGAGGU) in bacteria. The length of the 5'-UTR is 100 or more nucleotides long and the 3'-UTR is much longer, which is several kilobases long. In eukaryotes, there are reports on ribosome binding sequences that are part of the 5'-UTR. However, they do not have a fixed location like the Shine-Daigarno sequence, which is known as the ribosome binding site of the 5'-UTR in prokaryotes (Kozak M, 1987, Hamilton *et al.,* 1987, Yamauchi *ef al.,* 1991,Joshi *et al.,* 1997).

The amount of protein production depends on the translation efficiency of the mRNA, thus is important in gene regulation. The general function of 5'-UTR is to control mRNA translation using the contest of the neighboring sequence of the initiation codon. This is part of the 5'-UTR which is used to control the translation efficiency (Xiong W *et al*., 2001; Rogozin iB *et al.,* 2001; Gallie DR *et al.,* 1989; Mignone F *et al*., 2002; Kawaguchi R *et al.,* 2002, 2005). An example of 5'-UTR gene is the small subunit (RbcS) of Ribulose bisphosphate carboxylase/oxygenase (RUBISCO).

In order to find a method for improving translation efficiency to increase the production of the heterologous protein, the 5'-UTR sequences encoded in *A. thaliana* whole genome were searched for sequence homology with the 5'-UTR gene of the Ribulose bisphosphate carboxylase/oxygenase (RUBISCO) small subunit (RbcS). Fifty sequences with highest sequence similarities were screened. To detect the translation efficiency of screened sequences, 5' UTR::GFP structure was constructed. The translation efficiency of the GFP by each 5'-UTR was measured. The result indicated that out of 50 sequences screened, 6 of the 5'-UTR sequences showed the highest translation efficiency. These sequences were represented as SEQ ID NOs: 1 to 6.

In order to investigate the important role of 5'-UTR nucleotide sequence in protein translation efficiency, base substitution mutagenesis was performed on 5'-UTR of SEQ ID NO: 1. In detail, three nucleotides at the 3'-end of 5'-UTR of SEQ ID NO: 1 were substituted with continuous bases of either thymine (T), adenine (A), guanine (G) or cytosine (C). In addition, a mutant sequence whose last base at the 3'-end of 5'-UTR of SEQ ID NO: 1 substituted with thymine was generated. Also, mutant sequences were generated by substituting the base at position 4 or position 5 from the start codon of the heterologous protein, which is also the 3'-end region of 5'-UTR of SEQ ID NO: 1, with two continuous sequences of thymine (T), guanine (G) or cytosine (C). Also, a mutant sequence was generated by substituting the base at position 3 from the 3'-end of 5'-UTR of SEQ ID NO: 1 with thymine. The translation efficiency was measured for the above mutant UTR sequences.

All proteins showed translation efficiency higher than that of 5'-UTR of Rbc, except for a mutant sequence whose three bases at the 3'-end of 5'-UTR of SEQ ID NO: 1 substituted with continuous nucleotide of thymine (T), a mutant sequence whose one base at the end of 3'-end substituted with thymine, a mutant sequence whose base at position 3 from the 3'-end substituted with thymine and a mutant sequence whose base at position 4 and position 5 from the start codon substituted with thymine.

Furthermore, according to the above results, homology between the sequences with high protein translation efficiency was investigated. As a result, high incident of AAG or sequence similar to AAG were detected. Therefore, the present inventors generated an artificial sequence having AAG repeats which is 3 nucleotides at 3'-end of 5'-UTR of SEQ ID NO: 1. The protein translation efficiencies were higher than 5'-UTR Rbc control group.

Therefore, among the 50 of 5'-UTR sequences that were screened, 6 sequences which showed high translation efficiency and the mutant 5'-UTR sequence with high translation efficiency described above, were each represented by the following SEQ. ID NOs as shown in Table 4.

**[Table 4]**

| 5'-UTR | Nucleotide sequence | SEQ ID NOs |
|---|---|---|
| UTR1 | AGAGAAGACGAAACACAAAAG | 1 |
| UTR 2 | GAGAGAAGAAAGAAGAAGACG | 2 |
| UTR 6 | AAAACTTTGGATCAATCAACA | 3 |
| UTR 7 | CTCTAATGACCAGGAGTAAAA | 4 |
| UTR 24 | AGAAAAGCTTTGAGCAGAAAC | 5 |
| UTR35 | AACACTAAAAGTAGAAGAAAA | 6 |
| U1AAA | AGAGAAGACGAAACACAAAAA | 8 |
| U1CCC | AGAGAAGACGAAACACAACCC | 9 |
| U1GGG | AGAGAAGACGAAACACAAGGG | 10 |
| U1 (-4,5G) | AGAGAAGACGAAACACGGAAG | 13 |
| U1 (-4,5C) | AGAGAAGACGAAACACCCAAG | 14 |
| UAAG | AAGAAGAAGAAGAAGAAGAAG | 16 |

Therefore, the present invention provides a DNA fragment for improving translation efficiency of the heterologous protein place in the downstream, which comprises any one nucleotide sequence selected from the group consisting of SEQ ID NOs: 1∼6, SEQ ID NOs: 8∼10, SEQ ID NOs: 13∼14 and SEQ ID NO: 16.

Also, the present invention provides a recombinant vector containing the DNA fragment, which comprises any one nucleotide sequence selected from the group consisting of SEQ ID NOs: 1∼6, SEQ ID NOs: 8∼10, SEQ ID NO: 13∼14 and SEQ ID NO: 16.

In particular, the recombinant vector may use a conventional protein expression vector as a back bone, which comprises the DNA fragment operably linked to a promoter, and a polynucleotide encoding a heterologous protein.

As used herein, the term "expression vector" refers to plasmids, virus or other vehicles known in the art that has been manipulated by inserting or introducing the DNA fragment of the present invention and a polynucleotide encoding a heterologous protein. The DNA fragment and the polynucleotide encoding a heterologous protein may be operably linked to an expression control sequence. The operably linked polynucleotide and expression control sequence can be included in a single recombinant expression vector containing both a selection marker and a replication origin. As used herein, the term "operably linked" refers that the polynucleotide is linked to an expression control sequence in such a manner to enable the expression of a polynucleotide when a suitable molecule is bound to the expression control sequence. As used herein, the term "expression control sequence" refers to a DNA sequence that regulates the expression of the operably linked polynucleotide in a certain host cell. The expression control sequence include a promoter for performing transcription, an optional operator sequence for controlling transcription, a sequence corresponding to a suitable mRNA ribosome-binding site, and a sequence controlling termination.

The promoter is not specifically limited as long as it can overexpress the heterologous gene inserted into plants. Examples of the promoter include, but are not limited to, the 35S RNA and 19S RNA promoters of CaMV; a figwort mosaic virus (FMV) full-length transcript promoter, and the coat protein promoter of TMV. Vectors suitable to introduce the DNA fragment and polynucleotide encoding the heterologous protein into plant cells include Ti plasmids and plant virus vectors. Examples of the suitable vectors include, but are not limited to, binary vectors, such as pCHF3, pPZP, pGA and pCAMBIA series. Anyone skilled in the art can select a suitable vector for introducing the DNA fragment and polynucleotide encoding the heterologous protein. Any vector capable of introducing the DNA fragment and polynucleotide encoding the heterologous protein into the plant cell may be used.

The present inventors constructed an expression vector to induce a high level of protein expression in plants by using the DNA fragment capable of translating the heterologous protein with high efficiency and targeting the heterologous protein to a particular cellular organelle at the same time.

When protein heterologous protein is overexpressed in transgenic plants or in cells, proteolytic degradation of protein may occur. However, the protein can be stored more stable when the heterologous protein is targeted to particular cellular organelles. If the heterologous protein is targeted to ER, the proteolytic degradation can be minimized comparing to the localization in the cytosol. There is a report on 104-fold of increase in protein yield when the human growth factor was targeted to ER in tobacco plant (Wirth, S. *et al.,* 2004). There was a reduction of proteolytic degradation by using the ER retention signal peptide such as KDEL or HDEL, which increased the folding and assembly of the heterologous protein by molecular chaperon by retaining the heterologous protein in the ER (Nutall, J. *et al*., 2002). An example of 10∼100 fold increase in protein yield was reported by targeting a heterologous protein to ER instead of the secretory pathway (Hellwig, S. *et* a/., 2004).

Plant chloroplast is a good target for protein storage since it is an enormous protein storage place containing more than 40% of total soluble proteins and exists in high numbers. The plant chloroplast contains least amount of protease for protein processing, which prevents the heterologous protein from degradation, therefore being able to store protein in higher concentration.

Therefore, the present inventors generated a recombinant vector by further adding a leader polynucleotide inducing the targeting to a particular cellular organelle, such as ER or chloroplast to the recombinant expression vector containing a promoter, the DNA fragment for improving translation efficiency, and a polynucleotide encoding a heterologous protein in an operable manner,

When targeting the heterologous protein to ER in a plant cell, BiP (chaperone binding protein) may be used. Preferably, the genomic DNA of BiP containing the intron region represented by SEQ ID NO: 18 may be used instead of BiP cDNA. The nucleotide sequence encoding peptide HDEL (His-Asp-Glu-Leu) or KDEL (Lys-Asp-Glu-Leu) may be used for heterologous protein retention.

The BiP is a luminal binding protein and is identified with binding with immunoglobulin heavy chain binding protein and glucose regulated protein. BiP is located in ER and is one member of HSP70 chaperon family, and temporarily binds to the newly synthesized protein in the ER. The N-terminal of the BiP protein contains signal sequence for targeting the heterologous protein to ER.

In addition, when targeting the heterologous protein to the chloroplast of the plant cell, nucleotide sequence encoding Cab (chloroplast a/b binding protein) may be used.

The Cab (chloroplast a/b binding protein) contains a transit peptide for targeting to chloroplast. When using this peptide, the heterologous protein may be targeted normally to the chloroplast (Kavanagh TA. *et al.,* 1988). Therefore, when using an expression vector containing BiP signal sequence, ER retention signal or Cab transit peptide, they may affect the targeting the heterologous protein to ER or chloroplast and thus accumulating the heterologous protein in high levels for mass production.

Furthermore, the present invention provides a transgenic plant by introducing the recombinant vector of the present invention into a plant using any method among ones known in the art.

The method for introducing the recombinant vector into a plant includes, but is not limited to, *Agrobacterium* sp.-mediated transformation, particle gun bombardment, silicon carbide whiskers, sonication, electroporation and PEG (polyethylene glycol) precipitation. In another aspect of the present invention, the recombinant vector was introduced into *A. thaliana* by *Agrobacterium sp*.-mediated transformation method.

Moreover, the transgenic plant produced by introducing the recombinant vector according to the present invention, in which a heterologous protein is translated with high efficiency and accumulated in a particular cellular organelle, thereby enabling mass production of the heterologous protein with high efficiency may be produced by conventional sexual propagation or asexual propagation method known in the art. In particular, the plant according to the present invention may be produced by sexual propagation, which is a process of producing seed by pollination of the flower and reproducing from the seed. The asexual propagation of the selected transgenic plants can be performed through the processes of callus induction, rooting and soil acclimatization according to any method known in the art. Preferably, the explants of the plants transformed with the recombinant vector are placed in a suitable medium known in the art and then cultured in suitable conditions to induce the formation of calluses. When the shoots are formed, these shoots are transferred and cultured in a hormone-free medium. After about 2 weeks, the shoots are transferred to a rooting medium to induce rooting. The induced roots are transplanted and acclimated to soil, thus producing the plants. The transgenic plant of the present invention may include a whole plant and tissue, cell or seed produced by the plant.

The plant may be a dicotyledon or a monocotyledon. Examples of the dicotyledon include, but are not limited to, A. *thaliana,* soybean, tobacco plant, eggplant, red pepper, potato, tomato, Chinese cabbage, radish, cabbage, peach, pear, strawberry, oriental watermelon, melon, cucumber, carrot and celery. Examples of the monocotyledon include, but are not limited to, rice, wheat, barley, corn, sugar cane, oat and onion.

Furthermore, the present invention provides a method for producing a heterologous protein from a plant.

The heterologous protein that can be used in the present invention is a protein that may be produced by the genetic engineering method of the present invention, but is not limited thereto. Preferably, commercially useful proteins, that is, proteins with agricultural and pharmaceutical value and requiring mass-production may be included, but are not limited to, serum proteins (e.g., coagulation factors including Factors VII, VIII and IX), immunoglobulins, cytokine (e.g., interleukin), α-, β-and γ-interferons, colony stimulating, factors (e.g., G-CSF and GM-CSF), platelet-derived growth factor (PDGF), phospholipase activating protein (PLAP), insulin, tumor necrosis factor (TNF), growth factors (e.g., tissue growth factors and epithelial growth factors, such as TGF-α or TGF-β), hormones (e.g., follicle stimulating hormone, thyroid stimulating hormone, antidiuretic hormone, pigmentary hormone and parathyroid hormone, luteinizing hormone- releasing hormone and derivatives thereof), calcitonin, calcitonin gene related peptide (CGPR), enkephalin, somatomedin, erythropoietin, hypothalamic releasing factor, prolactin, chorionic gonadotropin, tissue plasminogen activator, growth hormone releasing peptide (GHPR) and thymic humoral factor (THF). Also, these proteins may include enzymes, which are exemplified by carbohydrate-specific enzymes, proteolytic enzymes, lipases, oxidoreductases, transferases, hydrolases, lyases, isomerases and ligases. A reporter protein is expressed by the reporter gene which helps to detect the protein activity in the cell through the protein label.

In another aspect of the present invention, green fluorescence protein (GFP) was used as the heterologous protein.

The method for producing a heterologous protein from a plant is accomplished by introducing the recombinant vector, in which the DNA fragment and a polynucleotide encoding the heterologous protein is operably linked to a promoter, into a plant or transforming into a plant cell; incubating for several hours in order to induce expression of the heterologous protein; and harvesting the expressed heterologous protein from the transgenic plant or the plant cell. Expression of the heterologous protein, may be performed according to any method known in the art. The heterologous protein, overexpressed in a transgenic plant or in transformed plant cells, can be recovered via various methods, such as isolation and purification, as well known in the art. Generally, in order to remove cell debris and the like, the medium containing the cell can be centrifuged, followed by precipitation such as salting-out (e.g., ammonium sulfate precipitation, sodium phosphate precipitation and the like) and solvent precipitation (e.g., protein fraction precipitation using acetone, ethanol and the like), or the like. Dialysis, electrophoresis or various types of column chromatographies can also be performed. The column chromatographies may include, for example, ion exchange chromatograph, gel-filtration chromatography, high performance liquid chromatography (HPLC), reverse-phase HPLC and affinity column chromatography, ultra filtration, which can .be performed alone or in combination (Maniatis et al., Molecular Cloning: A Laboratory Manual, Cold Spring Harbor Laboratory, Cold Spring Harbor, N.Y (1982); Sambrook et al., Molecular Cloning: A Laboratory Manual, 2d Ed., Cold Spring Harbor Laboratory Press (1989); Deutscher, M., Guide to Protein Purification Methods Enzymology, Vol. 182. Academic Press Inc., San Diego, Calif. (1990)).

In order to isolate and purify the heterologous protein from the transgenic plant more simple and faster, polynucleotide encoding for cellulose-binding domain (CBD), preferably, polynucleotide having nucleotide sequence represented as SEQ ID NO: 21 was additionally linked to the recombinant vector in an operable manner.

The recombinant vector containing the CBD may express the heterologous protein fused with CBD, which may be easily isolated by chromatography method using the cellulose as a carrier.

The CBD of the fusion heterologous protein may be leaved by appropriate enzyme digestion, followed by additional purification steps to generate non-fusion heterologous protein.

In one aspect of the present, when the recombinant vector containing a promoter, the DNA fragment for improving translation efficiency and a polynucleotide encoding a heterologous protein (GFP) was operably linked with CBD, the overexpressed heterologous protein was purified with a recovery rate of nearly 90% by using CBD. It was confirmed that when TEV protease recognition site was additionally linked to the recombinant protein containing CBD sequence, a CBD cleaved non-fusion protein was isolated after cleavage by TEV protease.

The present invention will now be described in further detail by examples. It would be obvious to those skilled in the art that these examples are intended to be more concretely illustrative and the scope of the present invention as set forth in the appended claims is not limited to or by the examples.

**<Example 1>**

**Screening of 5'-UTR sequence**

To improve the expression of the target gene, the present inventors used *A. thaliana* whole genome to screen the 5'-UTR with high translation efficiency. Sequences of the untranslated regions (UTRs) at the N-terminal regions of genes from *A. thaliana* whole genome were obtained using BLAST searches. Twenty one nucleotide sequences located in front of the start codon of the coding sequences were selected and then listed. Next, 21 nucleotide sequences from the 5'-UTR of encoding genes of *A. thaliana* whole genome were compared for sequence homology with the 5'-UTR sequence of Ribulose bisphosphate carboxylase/oxygenase (RUBISCO) small subunit (RbcS) gene, that is known for high translation efficiency. Sequences were selected by comparing the sequence homology with 5'-UTR of RbcS and grading the similarity from high to low.

**[Table 1]**

| The 5'-UTR sequences of 50 screened genes. | | |
|---|---|---|
| 5'-UTR NOs. | Nucleotide Sequences | Genes |
| 1 | AGAGAAGACGAAACACAAAAG | ubiquitin extension protein (UBQ1) |
| 2 | GAGAGAAGAAAGAAGAAGACG | UDP-glucose glucosyltransferase |
| 3 | AGAACGAAGTAGACGAAGACG | MAP kinase kinase 2 |
| 4 | GAGATTTAGAAGAAAAGGGAA | 3-phosphoinositide-dependent protein kinase-1 (PDK1) |
| 5 | GAGAGAAGAAGAATCGTGGAG | Gluthatione reductase, chloroplast precursor |
| 6 | AAAACTTTGGATCAATCAACA | xylulose kinase |
| 7 | CTCTAATCACCAGGAGTAAAA | ribosomal protein L32 |
| 8 | TTTTCATTGTCCTTGTGAAAA | peroxidase |
| 9 | ATCATCGGAATTCGGAAAAAG | casein kinase I |
| 10 | CGAATTATTCGCTAAAAAAAG | glucose-1 -phosphate denylyltransferase (APL3) |
| 11 | ACAAGCTAGAAACAAAGAAAC | putative cytochrome P450 |
| 12 | TGAAACTGAAGGAGAAGGAAG | dynamin-like protein 4 (ADL4) |
| 13 | AACGACAGATAGAGAGAAACG | chlorophyll a/b-binding protein |
| 14 | AGAGAGTGAGGGGGGAAGAAG | NADPH-ferrihemoprotein reductase ATR1 |
| 15 | GAAGAAGAAGAAGAAGGAAAG | cyclophilin - like protein cyclophilin |
| 16 | AGAGCCAAGAACAAAGAAACC | copper transport protein |
| 17 | AAACAAATCAAAGCAAAGATC | transcription factor |
| 18 | ACGCAAAGAAAACAGACCAAC | cysteine synthase |
| 19 | CAAAAGTAGTAACAACTAAGA | cytochrome P450 monooxygenase (CYP83A1) |
| 20 | GAGAGAGAGAGAGAGAGAGAG | MYB27 protein - like MYB27 protein |
| 21 | GCAAACAGAGTAAGCGAAACG | heat shock protein 17 |
| 22 | CCGCAGTAGGAAGAGAAAGCC | actin-like protein |
| 23 | CAAGGTAACAGATAAACACGA | hypothetical protein |
| 24 | AGAAAAGCTTTGAGCAGAAAC | translation releasing factor RF-2 |
| 25 | AAGGAGAGGAAGAAGAAGATC | FPF1 protein |
| 26 | TGTGTAACAACAACAACAACA | hypothetical protein |
| 27 | TGATTAGGAAACTACAAAGCG | subtilisin-like serine protease |
| 28 | AGAGACAAGAGAAGAGAGAGA | hypothetical protein |
| 29 | TATCTTTTTACGGATTTGAAG | unknown protein |
| 30 | GAGAGAGATCTTAACAAAAAA | Lil3 protein |
| 31 | GCGAAGAAGACGAACGCAAAG | ubiquitin extension protein (UBQ2) |
| 32 | AGAAGAAGAAGAAGAAGCAAA | photosystem I subunit V precursor |
| 33 | CCGAAGAGGAAGAAGAAGAAG | profilin 1 |
| 34 | AGGAAACTGAGGAACACAACA | microbody NAD-dependent malate dehydrogenase |
| 35 | AACACTAAAAGTAGAAGAAAA | Ca2+-dependent membrane-binding protein annexin |
| 36 | CTCAGAAAGATAAGATCAGCC | ubiquitin activating enzyme E1-like protein |
| 37 | AGAGAGTGACGGGGGAAGAAG | NADPH-ferrihemoprotein reductase ATR1 |
| 38 | AATAATAAGCCATTGAAAAAA | senescence-associated protein almost identical to ketoconazole resistant protein |
| 39 | TTACTTTTAAGCCCAACAAAA | cysteine proteinase |
| 40 | CAATTAAAAATACTTACCAAA | expansin-like protein expansin At-EXP6 |
| 41 | AACCAATCGAAAGAAACGAAA | putative protein heparanase |
| 42 | AAGACGGCAGTAACGAAGGCA | unknown protein |
| 43 | AAGAAGAAACAAAGAGAGAAG | unknown protein |
| 44 | AAAACAAAAGTTAAAGCAGAC | hypothetical protein |
| 45 | AAAGAAAGAGAGAGAGAGAGA | putative protein |
| 46 | GAACCAAGGAATAAAAGAAAA | putative anthocyanin 5-aromatic acyltransferase |
| 47 | GTTTTCCAAAGACAAACCAAC | cysteine synthase - like cysteine synthase |
| 48 | AGAAAAGCTTTGAGCAGAAAC | translation releasing factor RF-2 |
| 49 | GAAAGGCACACAAAATAACCC | putative RNA-binding protein |
| 50 | TTAGGACTGTATTGACTGGCC | unknown protein |

According to the result represented in Table 1, a total number of 50 A. *thaliana* 5'-UTR sequences demonstrating sequence similarity to RbcS 5'-UTR were selected, and then numbered from 1 to 50.

**<Examples2>**

**Selection of 5'UTR with high translation efficiency**

**<2-1> Construction of recombinant vector to confirm the translation efficiency of 5'-UTR**

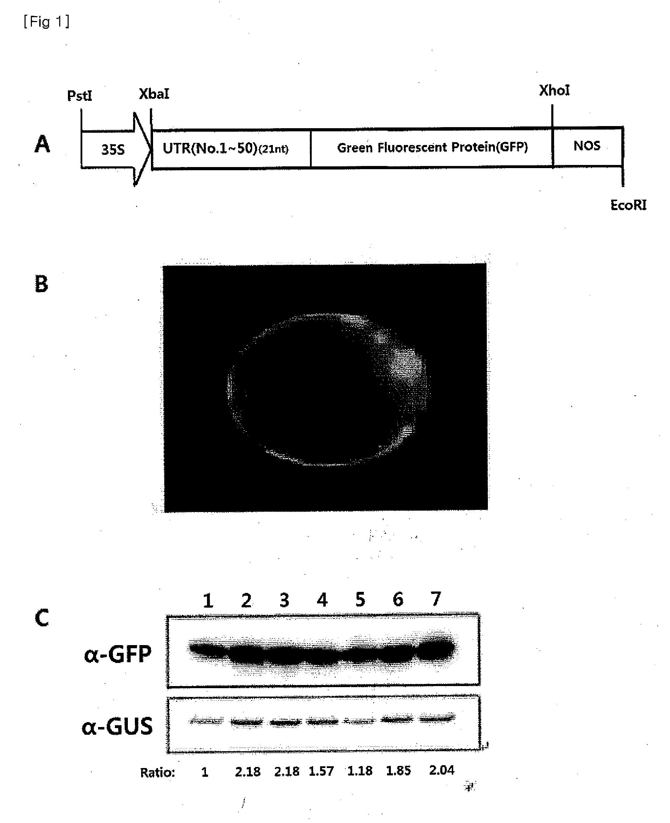

To determine the 5'-UTR sequence with the highest translation efficiency in *A. thaliana* protoplast, 5'-UTR::GFP structure was constructed by using PCR method for each of the 50 5'-UTR sequences screened from the Example 1. In detail, PCR amplification was performed by using plasmid 326-GFP as a template (*A. thaliana* Biological Resource center, Ohio State University, Ohio, USA). The 21 nucleotide sequences of 5'-UTR selected and the N-terminal region sequence of GFP including start codon AUG was used as a upstream primer. NOS terminator sequence was used as a downstream primer. DNA amplified by PCR was cloned into *Xcm*l-linearized pBluescript. After confirming the 5'-UTR and GFP cloning region by DNA sequencing, DNA encoding 5'-UTR-GFP region was digested with *Xba*l/*Xho*l, and then cloned into XhaIIXhoi-linearized 326-GFP3G (326-GFP vector with *Sma*I/*Hin*dIII/*Cla*I/*Sal*I/*Xho*I restriction enzyme sites added in front of the nos terminator) to generate a plasmid containing 35S promoter (35Sp) of cauliflower mosaic virus, UTR::GFP and nos terminator (see Figure 1a.). As a control group for the following experiments, a vector containing RbcS 5'-UTR sequence instead of the 5'-UTR screened sequences was used.

**<2-2> Selection of 5'-UTR with high translation efficiency using immunofluorescence microscopy and immunoblotting**

The plasmids 35Sp-UTR::GFP containing each of the 50 5'-UTR sequences were introduced into *A. thaliana* protoplast by the PEG-mediated transformation method known in the art (Jin er al., Plant Cell 13: 1511-1526, 2001). Expression of the GFP in the cytosol from each 5'-UTR sequence was monitored by using fluorescence microscopy. In addition, the translation efficiency of the 5'-UTR was confirmed by immunoblotting method. The *A. thaliana* leaf transformed with the 35Sp-UTR::GFP plasmid was kept for 24 hrs in a 23°C incubator. Following incubation, the storage solution, W5 medium (154 mM NaCl, 125 mM CaCl₂, 5 mM KCI, 5 mM Glucose, 1.5 mM MES, pH 5.6) was removed. Protein extraction was performed by adding the denaturation buffer (containing 10% SDS and β-mercaptoethanol) to lysis the protoplast and then heated in a boiling water bath. The protein extract was centrifuged at 13,000 rpm for 10 min at 4°C to separate the pellet and the supernatant. The isolated supernatant was separated by SDS-PAGE, and immunoblotted with monoclonal anti-GFP antibody (Clontech cat no. 632381) to detect the protein expression of 35Sp-UTR(NO. 1∼50)::GFP..

According to the result represented in Figure 1b, the immunofluorescence microscopy analysis confirmed that the 5'-UTR of the present invention showed various expression efficiencies without affecting the natural characteristics of GFP. In particular, as shown from the immunoblotting result of Figure 1c, of the 50 5'-UTRs screened, 5'-UTR NO. 1, 2, 6, 7, 24 and 36 showed about one to two-folds increase in the expression level of GFP when compared to the control, 5'-UTR of RbcS.

**[Table 2]**

| Five 5'-UTR sequences with improved translation efficiency | | |
|---|---|---|
| 5'-UTR NOs | Nucleotide sequences | SEQ ID NOs |
| 1 | AGAGAAGACGAAACACAAAAG | 1 |
| 2 | GAGAGAAGAAAGAAGAAGACG | 2 |
| 6 | AAAACTTTGGATCAATCAACA | 3 |
| 7 | CTCTAATCACCAGGAGTAAAA | 4 |
| 24 | AGAAAAGCTTTGAGCAGAAAC | 5 |
| 35 | AACACTAAAAGTAGAAGAAAA | 6 |

**<Example 3>**

**Analysis of translation efficiency by base substitution of 5'UTR**

**<3-1> Analysis of translation efficiency of mutant 5'UTR**

According to the result of Example 2, the present inventors confirmed that of the 50 5'-UTRs screened, 5'-UTR NOs: 1, 2, 6, 7, 24 and 36 showed high translation efficiency. In order to investigate the importance of 5'-UTR base in translation efficiency of 5'-UTR, an experiment was performed to analyze the translation efficiency of the 5'-UTR with base substitution. First, base substitution mutagenesis UTR was generated for 5'-UTR NO. 1 (SEQ ID NO: 1), which was show to have high translation efficiency. In detail, three bases at the 3'-end of 5'-UTR NO: 1 were substituted with continuous bases of either thymine (T), adenine (A), guanine (G) or cytosine (C). In addition, a plasmid having the last base at the 3'-end of the 5'-UTR of SEQ ID NO: 1 substituted with thymine was constructed.

Also, mutant sequences whose base at position 4 and position 5 from the start codon of GFP, which is also the 3'-end region of 5'-UTR NO: 1 substituted with two continuous sequences of T, G or C were generated. Mutant sequences whose third base from the 3'-end of the 5'-UTR of substituted with thymine were constructed. The UTR sequence of mutants in which part of their 5'-UTR of SEQ ID NO: 1 nucleotide sequence is substituted, are shown in Table 3. Next, a 5'-UTR mutant::GFP plasmid was generated by fusing mutant UTRs with GFP. Construction of the plasmid was performed similar to the method described in the Example <2-1>. The constructed plasmid was transferred into the protoplast of *A. thaliana* via PEG-mediated transformation. Protein was extracted following the method described in the Example <2-2>. The supernatant was separated by SDS-PAGE, and then immunoblotted with monoclonal anti-GFP antibody (Clontech cat no. 632381) to analyze the expression of 35Sp-UTR (mutant)::GFP protein. As a control, RbcS 5'-UTR (SEQ ID NO: 17: 5'-CACAAAGAGTAAAGAAGAACA-3') was used instead of the 5'-UTR sequence.

**[Table 3]**

| Sequences of 5'-UTR mutants derived from base substitution of UTR of SEQ ID NO:1 | | |
|---|---|---|
| 5'-UTR | Nucleotide sequence | SEQ ID NOs |
| 5'-UTR NO: 1 | AGAGAAGACGAAACACAAAAG | 1 |
| U1 TTT | AGAGAAGACGAAACACAATTT | 7 |
| U1 AAA | AGAGAAGACGAAACAGAAAAA | 8 |
| U1 CCC | AGAGAAGACGAAACACAACCC | 9 |
| U1 GGG | AGAGAAGACGAAACACAAGGG | 10 |
| U1 (-1T) | AGAGAAGACGAAACACAAAAT | 11 |
| U1 (-4,5T) | AGAGAAGACGAAACACTTAAG | 12 |
| U1 (-4,5G) | AGAGAAGACGAAACACGGAAG | 13 |
| U1 (-4,5C) | AGAGAAGACGAAACACCCAAG | 14 |
| U1 (-3T) | AGAGAAGACGAAACACAATAG | 15 |
| UAAG | AAGAAGAAGAAGAAGAAGAAG | 16 |

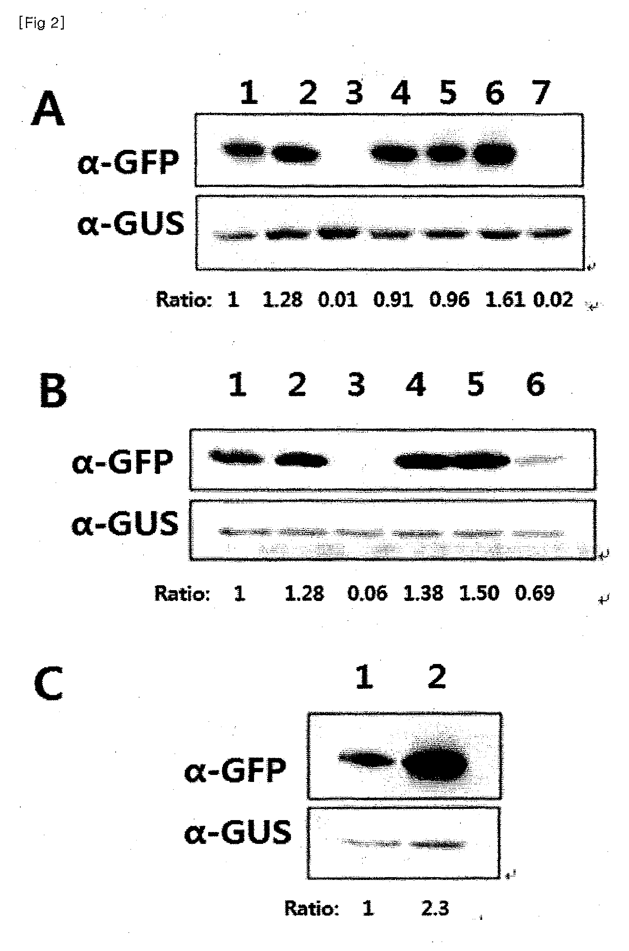

Therefore, according to the result represented in Figure 2a, when considering the expression level of control RbcS 5'-UTR as 1, the mutants of SEQ ID NO: 7, 35Sp-U1TTT:GFP and SEQ ID NO: 11, 35Sp-U1 (-1T)::GFP showed almost no expression. 35Sp-U1AAA::GFP (SEQ ID NO: 8) showed 0.91-fold, 35Sp-U1CCC::GFP (SEQ ID NO: 9) showed 0.96-fold higher expression than control, while 35Sp-U1GGG::GFP (SEQ ID. NO: 10) showed 1.6-fold higher level of expression compared to the control group.

In addition, as shown in Figure 2b, there was almost no or very low expression of GFP in UTR mutants of SEQ ID NO: 12 and SEQ ID NO: 15. However, UTR mutants of SEQ ID NO: 13 and SEQ ID NO: 14 showed increased level of expression when compared to the control group.

**<3-2> Analysis of translation efficiency of mutant 5'UTR with repeated AAG**

When the 5'-UTR sequences from result of Example <3-1> showing high translation efficiency were compared to each other, a high incident of AAG or sequence similar to AAG were detected. Therefore, to analyze the effect of AAG sequence in translation efficiency, the present inventors generated 5'-UTR with repeated AAG as represented by SEQ ID NO: 16 in Table 4. The construct was used to transform *A. thaliana* as described previously in <3-1>, and the amount of GFP expression was analyzed by immunoblotting.

According to the result shown in Figure 2c, when the 5'-UTR contained AAG repeats, there was approximately 2-fold higher level of GFP expression when compared to the control group.

**<Examples 4>**

**Analysis of translation efficiency of 5'-UTR in monocotyledon plant**

The result of Example 3 indicated that the 5'-UTR with bases sequences of SEQ ID NOs: 1 to 17 increased the translation efficiency in the dicotyledon plant, *A. thaliana.* Therefore, an *in vitro* translation experiment was performed to confirm using 5'-UTR of SEQ NO: 1, whether the 5'-UTR sequence of the present invention can increase the protein translation in monocotyledon plant by using wheat germ extract system (Promega, TNTT7 Coupled Wheat Germ Extract System, cat no. L4140). For this experiment, 35Sp-RbcUTR::GFP (control) and 35Sp-UTR1::GFP cloned in 326-GFP3G were each digested with *Xba*l/*Xh*ol and cloned into pBluescript linearized with *Xba*l/*Xho*l. This was linearized again with restriction enzyme *Xmn*I to construct T7p-RbcUTR::GFP and T7p-UTR1::GFP plasmids that uses T7 promoter (T7p). Protein extraction and immunoblotting was performed according to the method previously described.

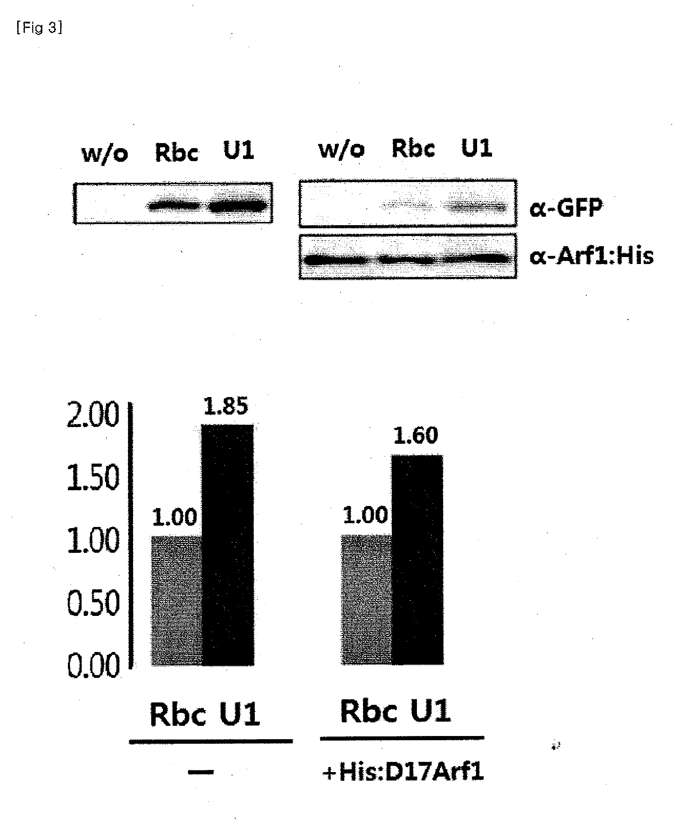

According to the result shown in Figure 3, in the case of UTR1 of the present invention, the expression amount of GFP was about 2-fold higher than that of the control group.

Therefore, the result indicates that the UTR sequences of the present invention, that is the UTRs with nucleotide sequences represented by SEQ ID NO: 1∼6, SEQ ID NO: 8∼10, SEQ ID NO: 13∼14 and SEQ ID NO: 16 can increase the protein translation of in both *in vivo* and *in vitro* conditions. The increase of protein translation in monocotyledon plants as well as in dicotyledon plants such as A. *thaliana,* suggests that UTR mediated protein translation mechanism are conserved in both dicotyledon and monocotyledon plants.

**<Example 5>**

**Construction of ER targeting expression vector containing the 5'-UTR sequence**

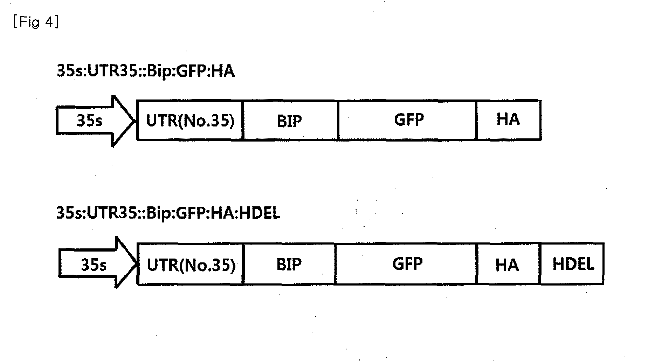

The present inventors used the leader sequence of BiP (chaperone binding protein) as represented by SEQ ID NO: 18 to construct an expression vector which can target the protein of interest to ER and expresses the protein in high levels. The BiP sequence of the SEQ ID NO: 18 encodes 14 amino acid sequence. To improve the translation efficiency of the protein, whole genomic DNA containing intron instead of cDNA was used. In addition, HDEL peptide sequence, which is well known to localize the foreign protein for longer durations inside ER was used for the vector construction. As for the UTR sequence, UTR of SEQ ID NO: 6 showing high protein translation efficiency was used. In summary, the present inventors constructed expression vectors, 326-35Sp-UTR35::BiP:GFP:HA and 326-35Sp-UTR35::BiP:GFP:HA:HDEL, as shown in Figure 4 for targeting the protein to ER. For the vector construction, plasmid 326-GFP (A. *thaliana* biological Resource center, Ohio State University, Ohio, USA) was used as the basic vector. UTR sequence of SEQ ID NO: 6 was cloned behind 35S promoter and green fluorescent protein (sGFP, Clontech, USA) with HA tagging was used to generate a chimeric structure. Next, the N-terminal region (BiP sequence of SEQ ID NO:18) containing the signal sequences of chaperon binding protein (BiP) was fused to the N-terminal region of the GFP-coding region to generate 35Sp-UTR35::BiP:GFP:HA. In addition, 4 amino acids, HDEL were linked at the C-terminal of HA tag in 35Sp-UTR35::BiP:GFP:HA to generate 35Sp-UTR35::BiP:GFP:HA:HDEL. The BiP insert was PCR amplified by using primer pairs of SEQ ID NO: 19 and SEQ ID NO: 20. The sequence of SEQ ID NO:18 was used as a template, and then fused to the N-terminnal of the GFP coding region of the basic vector to generate 35Sp-UTR35::GFP:HA or 35Sp-UTR35::GFP:HA:HDEL.

SEQ ID NO: 19 (BIP-F): 5'-ATGGCTCGCTCGTTTGGAGCT-3'

SEQ ID NO: 20 (BIP-R): 5'-TAACTTCGTAGCCTCTTCTATTG-3'

**<Example 6>**

**Detection of protein localization in the ER by ER targeting**

### expression vector

The vectors, 326-35Sp-UTR35::BiP:GFP:HA and 326-35Sp-UTR35::BiP:GFP:HA:HDEL constructed from the Example 5 were introduced into the protoplast of *A. thaliana* via PEG-mediated transformation method of Example <2-2> to generate a transgenic *A. thaliana.* Next, the localization of GFP inside the cells of transformed *A. thaliana* leaf was analyzed by using immunofluorescence microscopy method of Example <2-2>.

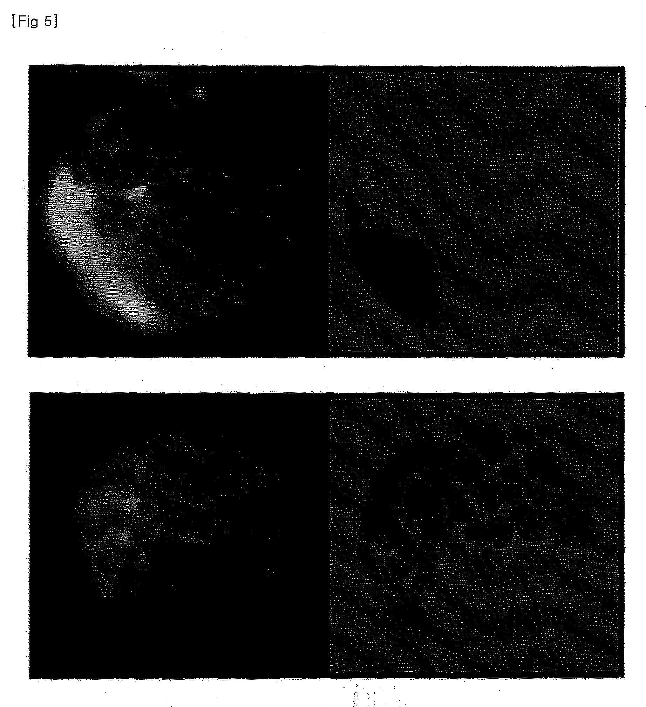

As shown in Figure 5, GFP proteins were localized in ER of the A. *thaliana* transformed with 35Sp-UTR35::BiP:GFP:HA or 35Sp-UTR35::BiP:GFP:HA:HDEL vectors. Therefore, the result suggested that the vector of the present invention is capable of targeting the heterologous protein to the ER.

**<Example 7>**

**Construction of a vector for isolating and purifying the protein expressed in the plant**

The present inventors constructed a vector for isolating and purifying the overexpressed and highly translated protein from the plant according to the result from Example 6. Cellulose binding domain (CBD) was used as a tag for purifying the protein, and a TEV protease cleavage site was used to remove this domain. In detail, using 326-35Sp-UTR35::BiP:GFP:HA and 326-35Sp-UTR35::BiP:GFP:HA:HDEL prepared from Example 5 as basic vectors, the substrate site of the TEV protease and the CBD domain were cloned to generate vectors 326-35Sp-UTR35::BiP:GFP:HA:TEV:CBD and 326-35Sp-UTR35::BiP:GFP:HA:TEV:CBD:HDEL. When constructing the above vectors, the substrate site of the TEV protease known in the art and the nucleotide sequence coding the CBD domain of SEQ ID NO: 21 were used as a template. After PCR amplification using primer pairs of SEQ ID NO: 22 and SEQ ID NO: 23, the PCR product was each cloned into C-terminal of HA tag in 326-35Sp-UTR35::BiP:GFP:HA and 326-35Sp-UTR35::BiP:GFP:HA:HDEL plasmids to generate 326-35Sp-UTR35::BiP:GFP:HA:TEV:CBD vector and 326-35Sp-UTR 35::BiP:GFP:HA:TEV:CBD:HDEL vector, respectively.

SEQ ID NO: 22 (CBD F primer): 5'-TTACACATGGCATGGATGAACT-3'

SEQ ID NO: 23 (CBD R primer): 5'-CTAAGTTCTTGATTTGAGAATAC-3'

**<Example 8>**

**Analysis of protein expression and accumulation after introducing protein isolation and purification vector into the plant**

The level of protein expression and accumulation was analyzed in the cellular organelles of plants using the vectors, 326-35Sp-UTR35::BiP:GFP:HA:TEV:CBD and 326-35Sp-UTR35::BiP:GFP:HA:TEV:CBD:HDEL prepared in Example 7. As a control, 35Sp-UTR22::GFP vector was, used. The vectors were transformed into the protoplast isolated from the A. *thaliana* leaf, and then incubated at 23°C for 24 hr or 48 hr. Proteins were isolated from the protoplast by adding protein extraction solution (10 mM HEPES pH 7.5, 10 mM NaCl, 3 mM MgCl₂, 5 mM DTT, 5mM EDTA, 5mM EGTA 0.2 % Triton X-100, protease inhibitor cocktail) before incubating for 1 hr at 4°C. The protein extract solution was centrifuged at 13,000 rpm for 10 min to separate the supernatant and the precipitate. The supernatant was separated by SDS-PAGE followed by Western blotting using monoclonal anti-GFP antibody (Clontech cat no. 632381) to analyze the amount of protein expression.

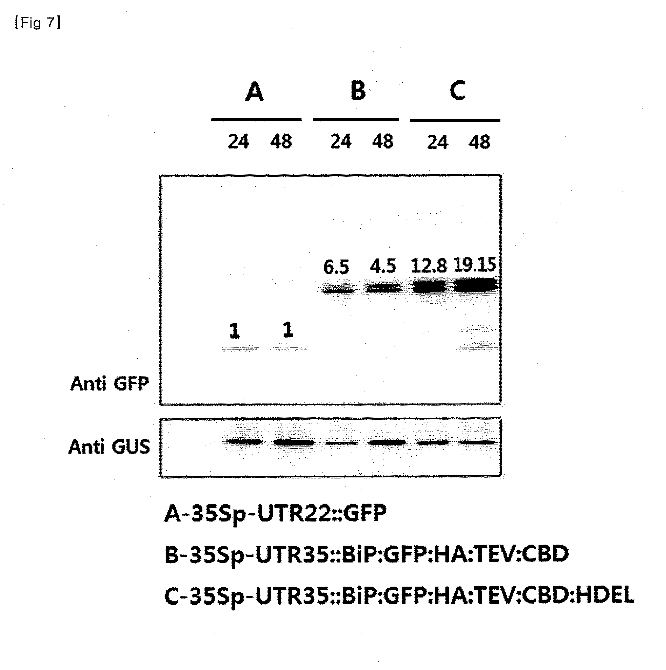

As shown in Figure 7, higher signals were detected in experimental groups treated with vectors 326-35Sp-UTR35::BiP:GFP:HA:TEV:CBD and 326-35Sp-UTR35::BiP:GFP:HA:TEV:CBD:HDEL, when compared to the control group. In addition, when control signal was considered as 1, there was a 6.5-fold and 12.8-fold increase of protein expression at 24 hr and 4.5-fold and 19.15-fold increase at 48 hr, when transformed with 326-35Sp-UTR35::BiP:GFP:HA:TEV:CBD and 326-35Sp-UTR35::BiP:GFP:HA:TEV:CBD:HDEL, respectively.

Evidence is provided from the result that the vector of the present invention for isolating and purifying DNA can improve the expression and accumulation of the heterologous protein stably for extended periods of time.

**<Example 9>**

**Generation of transgenic plants**

Transgenic plants were generated to analyze 1) the effect on increase of protein production by the UTR sequence of the present invention and the sequence targeting protein to specific cells; 2) the effect on protein isolation and purification by using CBD tag which was proven to improve protein expression by UTR without affecting the protein targeting. The plasmids for plant transformation was generated by digesting the 35S promoter and nos terminator region from the 326-35Sp-UTR35::BiP:GFP:HA, 326-35Sp-UTR35::BiP:GFP:HA:HDEL, 326-35Sp-UTR35::BiP:GFP:HA:TEV:CBD and 326-35Sp-UTR35::BiP:GFP:HA:TEV:CBD:HDEL plasmid. The DNA regions were cloned into the multiple cloning site of pCAMBIA1300 and pCAMIA2300, respectively, by using Pstl and EcoRl restriction enzymes. As a result, the following recombinant vectors, pCAMBlA1300-35Sp-UTR35::BiP:GFP:HA, pCAMBIA2300-35Sp-UTR35:BiP:GFP:HA:HDEL, pCAMBIA1300-35Sp-UTR35::BiP:GFP:HA:TEV:CBD, and pCAMBIA2300-35Sp-UTR35:BiP:GFP:HA:TEV:CBD:HDEL were generated for transformation. The transgenic plants harboring the recombinant vector were generated by *Agrobacterium-*mediated transformation method. The basic vector, pCABIA1300 of the transformation vector used hygromycin as the selection marker in the plant, and pCABIA2300 underwent Kanamycin resistance test to select the transgenic plants transformed with recombinant vector. The transgenic plants selected by antibiotics resistance testing were analyzed for the expression of these constructs by Western blotting to obtain T₁ generation. In the T₂ generation, if the death: survival ratio was 1:3 for the selection marker, this transgenic line was considered as single copy. This plant line is referred to as T₃, and the T₃ generation resistant to antibiotics and expressing protein were considered as homo, thereby a transgenic plant line was obtained.

**<Example 10>**

**Isolation and purification of target protein from the plant using the_ vector**

**<10-1> Isolation and purification of protein from transformed A. *thaliana* protoplast**

The present inventors isolated and purified overexpressed protein from the plant using the vector of the present invention. In detail, among the vectors prepared from the above mentioned examples, 326-35Sp-UTR35::Bip:GFP:HA:TEV:CBD plasmid was introduced into *A. thaliana* protoplast by PEG-mediated transformation (Jin et al:, Plant Cell, 13:1511-1526, 2001). Later, Bip:GFP:HA:TEV:CBD protein expressed in protoplast of the *A. thaliana* was isolated and then analyzed by Western blotting, to check the purity of 35Sp-UTR35::Bip:GFP:HA:TEV:CBD protein and whether 35Sp-UTR35::Bip:GFP:HA and CBD were separated by treating with TEV protease. In detail, the protein extracts from the *A. thaliana* protoplast transformed with 35Sp-UTR35::Bip:GFP:HA:TEV:CBD was treated with extraction buffer (10 mM HEPES pH 7.5, 10 mM NaCl, 3 mM MgCl₂, 5 mM DTT, 5mM EDTA, 5mM EGTA 0.2 % Triton X-100, protease inhibitor cocktail) and then the solution was centrifuged at 13,000 rpm for 10 min to separate the supernatant and the precipitate. The supernatant was then mixed with pretreated 0.3∼0.5% cellulose beads (Sigma S3504 Cellulose Type 20)and then incubated for 3∼4 hrs at 4°C before packing the cellulose beads in a Poly-Prep chromatography column (Bio-Rad, USA). Nonspecific proteins bound to the cellulose beads were removed by flowing 5 ml of protein extract solution. The cellulose beads were transferred to a new tube and incubated for 10∼30 min with an elution buffer containing 1.6% of cellobios. The solution was centrifuged at 13,000 rpm at 4°C to precipitate the cellulose beads. The supernatant containing purified 35Sp-UTR35::Bip:GFP:HA:TEV:CBD protein was collected for further experiment. Since 35Sp-UTR35::Bip::GFP:HA:TEV:CBD protein contains amino acid sequence cleaved by TEV protease, cleavage by TEV protease was analyzed. This was performed by treating purified 35Sp-UTR35::Bip:GFP:HA:TEV:CBD protein with TEV protease for 6 hrs at 4°C, separating by SDS-PAGE and then immunoblotting using monoclonal anti-GFP antibody (Clontech cat no. 632381).

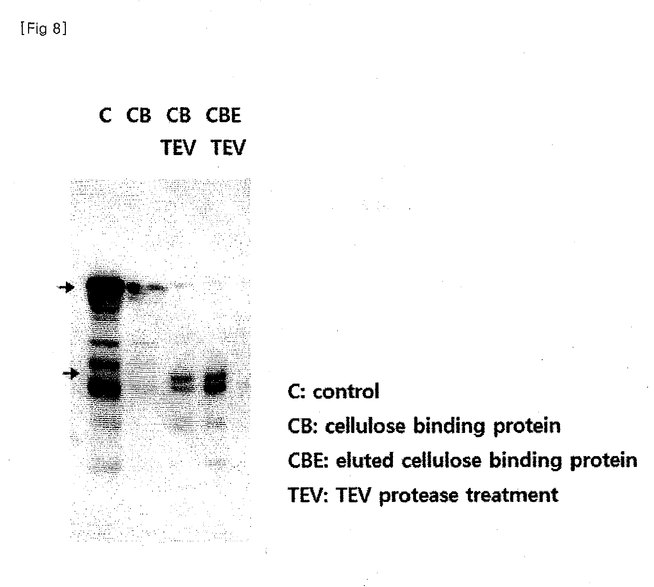

Based on the result shown in Figure 8, TEV protease were able to cleave the 35Sp-UTR35::Bip:GFP:HA:TEV:CBD protein that was expressed. In non TEV protease treated groups, 35Sp-UTR35::Bip:GFP:HA:TEV:CBD protein was detected at their expected full size, but in TEV protease treated groups, a band size of 35Sp-UTR35::Bip:GFP:HA protein without CBD was detected.

**<10-2> Isolation and purification of Protein from the transgenic plant**

According to the result from Example <10-1>, the vector encoding CBD enabled the isolation and purification of the heterologous protein, and also the TEV enzyme was able to cleave the correct cleavage site in the protoplast: Therefore, the present inventors analyzed whether it was possible to isolation and purify the CBD protein in transgenic plants introduced with CBD encoding vector. Protoplast of T₃ transgenic plant of 35Sp-UTR35::BiP:GFP:CBD:HDEL were used for the experiment under same buffer conditions. In detail, supernatant containing the purified 35Sp-UTR35::BiP:GFP:CBD:HDEL was collected and the protein was confirmed by Coomassie staining. The purity of the protein was confirmed by Western blotting using GFP antibody.

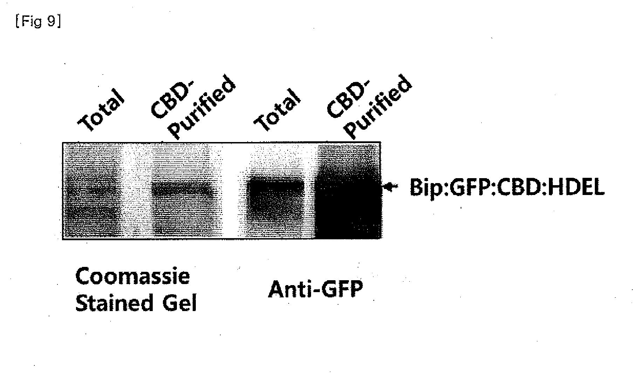

From the result shown in Figure 9, the purification yield of 35Sp-UTR35::BiP:GFP:CBD:HDEL protein expressed in the transgenic plants by using CBD was almost 90%.

Therefore, from the above result, the present inventors confirmed that UTR induced translation of heterologous proteins in cellular organelles at high efficiency, and CBD provided simple and convenient purification method of the heterologous protein at high yield.

**<Example 11>**

**Analysis of the level of gene expression at transcription and translational steps by the UTR sequence**

The UTR sequence selected from the present invention was confirmed to increase the amount of protein expressed in plants. Therefore, to understand whether the observed increase in protein expression of the different UTR sequences were due to a difference in the amount of gene transcript from DNA to mRNA, or the amount of gene transcript is similar but differentially regulated at the protein translation level, the gene expression level of 35Sp-UTR::GFP was analyzed at transcriptional and translational level. For this study, the following plasmids 35Sp-RbcUTR::GFP, 35Sp-UTR1::GFP, 35Sp-UTR35::GFP and a plasmid whose 3 bases at the C-terminal regions of the UTRs of SEQ ID NO: 1 and SEQ ID NO: 35 are substituted with thymine were generated. The construction of U1TTT::GFP and U35TTT::GFP plasmids were performed according to the method previously described. The vectors were transformed into the protoplast of *A. thaliana* and further incubated at 23°C for 24 hrs. The samples for analyzing transcription and translation levels were collected using the appropriate methods. Protein extraction was performed according to the method described in Example <2-2>. The expression level of UTR::GFP protein was analyzed by SDS-PAGE followed by immunoblotting with monoclonal anti-GFP antibody (Clontech cat no. 632381).

In addition, RT-PCR was performed to investigate the expression of UTR::GFP protein at the transcriptional level. First, RNA was extracted from the protoplast transformed with the vectors using TRIZOL Reagent (Invitrogen, Cat no. 15596-026) method. As a control, 18S ribosomal RNA and GUS were used. cDNA was generated from 2 µg of total RNA using the Superscript™ II Reverse Transcriptase (Invitrogen, Cat. No. 18064-022) PCR was performed with ExTaq (TaKaRa, Cat no.RR001A). The primers that were used for detecting transcription of GFP are represented by SEQ ID NO: 24 (5'-AGTTGTCGCAATTGTTGTTG-3') and SEQ ID NO: 25 (5'-CTGCCATGATGTATACGTTG-3'). For the detection of 18S ribosomal RNA, primers represented by SEQ ID NO: 26 (5'-ATGATAACTCGACGGATCGC-3') and SEQ ID NO: 27 (5'-CCTCCAATGGATCCTCGTTA-3') were used. For the detection of GUS, primers represented by SEQ ID NO: 28 (5'-AGTGTGGGTCAATAATCAGG-3') and SEQ ID NO: 29 (5'-CTGTGACGCACAGTTCATAG-3') were used.
The PCR condition for GFP was annealing at 50°C, 18S ribosomal RNA at 48°C, and GUS at 55°C, respectively.

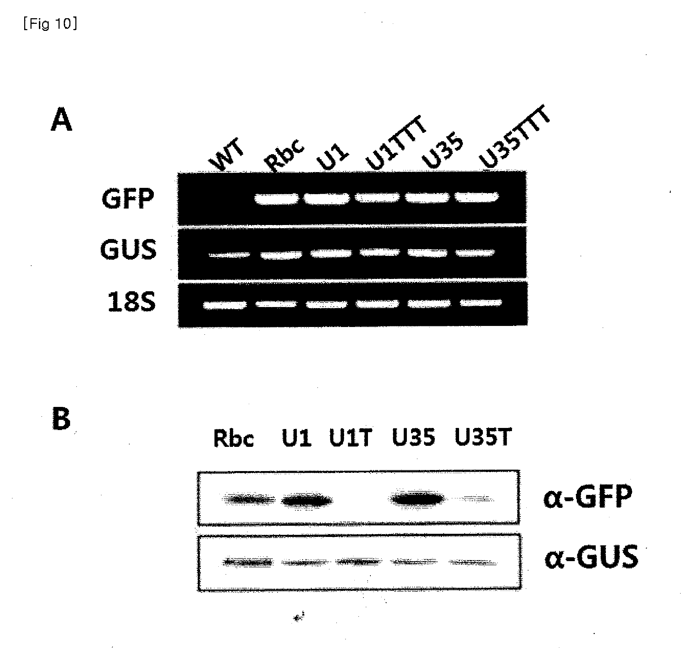

According to the result shown in Figures 10a and 10b, there was no difference at the transcriptional level of 35Sp-RbcUTR::GFP, 35Sp-UTR1::GFP, 35Sp-UTR1TTT::GFP, 35Sp-UTR35::GFP and 35Sp-UTR35TTT::GFP. However, there was a difference in the expression level of proteins. The 5'-UTRs of SEQ ID NO: 1 and SEQ ID NO: 35 showed significantly higher increase of protein translation when compared to the control group. There was an inhibition of protein translation when the 3'-termial of UTR was substituted with thymine.

Therefore, the present inventors observed that even if the transcript amount was similar, there was a different level of protein expression by the 5'-UTR sequences, therefore suggesting a possible regulation at the protein translation step, and not by the difference in the transcript amount from DNA to mRNA.

The above-disclosed subject matter is to be considered illustrative, and not restrictive, and the appended claims are intended to cover all such modifications, enhancements, and other embodiments, which fall within the true spirit and scope of the present invention. Thus, to the maximum extent allowed by law, the scope of the present invention is to be determined by the broadest permissible interpretation of the following claims and their equivalents, and shall not be restricted or limited by the foregoing detailed description.

## Claims

1. A DNA fragment for improving translation efficiency of a heterologous protein placed in the downstream, which comprises any one nucleotide sequence selected from the group consisting of SEQ ID NOs: 1∼6, SEQ ID NOs: 8∼10, SEQ ID NOs: 13∼14 and SEQ ID NO:16.

2. A recombinant vector containing a DNA fragment for improving translation efficiency, which comprises any one nucleotide sequences selected from the group consisting of SEQ ID NOs: 1∼6, SEQ ID NOs: 8∼10, SEQ ID NOs: 13∼14 and SEQ ID NO: 16.

3. The recombinant vector of claim 2, wherein the DNA fragment is operably linked to a promoter, and a polynucleotide encoding a heterologous protein.

4. The recombinant vector of claim 3, wherein the DNA fragment is operably linked to an additional polynucleotide for targeting or retaining the heterologous protein to ER of a plant cell.

5. The recombinant vector of claim 4, wherein the polynucleotide for targeting the heterologous protein to ER of the plant cell is a polynucleotide encoding BiP (chaperone binding protein) represented by SEQ ID NO: 18, and the polynucleotide for retaining the heterologous protein is a polynucleotide encoding peptide HDEL (His-Asp-Glu-Leu) or KDEL (Lys-Asp-Glu-Leu).

6. The recombinant vector of claim 4, wherein, the DNA fragment is further linked to a polynucleotide encoding cellulose-binding domain (CBD) represented by SEQ ID: 21 in an operable manner.

7. A method for mass production of a heterologous protein from a plant, which comprises a step of introducing the recombinant vector according to any one of claims 2 to 6 into the plant.

8. The method of claim 7, wherein the introducing the recombinant vector into a plant is performed using any one selected from the group consisting of *Agrobacterium* sp.-mediated transformation, particle gun bombardment, silicon carbide whiskers, sonication, electroporation and PEG (polyethylene glycol) precipitation.

9. The method of claim 7, wherein the plant is dicotyledon selected from the group consisting of *A. thaliana,* soybean, tobacco plant, eggplant, red pepper, potato, tomato, Chinese cabbage, radish, cabbage, peach, pear, strawberry, watermelon, oriental melon, cucumber, carrot and celery; or monocotyledon selected from the group consisting of rice, barley, wheat, rye, corn, sugar cane, oat and onion.
